# EUROPEAN PATENT SPECIFICATION

(11) **EP 4 516 331 B1**
(45) Date of publication and mention of the grant of the patent: **09.09.2026**
(21) Application number: 23842026.9
(22) Date of filing: 27.06.2023
(51) Int. Cl.: A61L 31/14, A61L 31/12, A61L 31/18, A61F 2/04

(54) **DEGRADABLE URETERAL STENT AND PREPARATION METHOD THEREFOR**
ABBAUBARER HARNLEITERSTENT UND HERSTELLUNGSVERFAHREN DAFÜR
STENT URÉTÉRAL DÉGRADABLE ET SON PROCÉDÉ DE PRÉPARATION

(30) Priority: 19.07.2022 CN 202210848552
(43) Date of publication of application: 05.03.2025
(73) Proprietor: Zhejiang Zhongzai Medical Technology Co., Ltd., Yiwu Jinhua, Zhejiang 322000 (CN)
(72) Inventor: CHEN, Hechun, Chengdu, Sichuan 610041 (CN); BAO, Yihong, Jinhua, Zhejiang 322000 (CN); CHEN, Dongliang, Chengdu, Sichuan 610041 (CN); BAO, Yiliang, Jinhua, Zhejiang 322000 (CN); ZHANG, Tianyao, Chengdu, Sichuan 610041 (CN); JIN, Xin, Jinhua, Zhejiang 322000 (CN); CHEN, Long, Chengdu, Sichuan 610041 (CN); ZHOU, Tong, Jinhua, Zhejiang 322000 (CN); XIAO, Jianping, Chengdu, Sichuan 610041 (CN); ZHU, Xiaozhen, Jinhua, Zhejiang 322000 (CN); WANG, Xin, Chengdu, Sichuan 610041 (CN); XU, Xiuzhong, Jinhua, Zhejiang 322000 (CN); ZHANG, Zhiping, Chengdu, Sichuan 610041 (CN); WU, Fan, Jinhua, Zhejiang 322000 (CN); XIONG, Chengdong, Chengdu, Sichuan 610041 (CN)
(74) Representative: karo IP
(86) International application number: PCT/CN2023/102900
(87) International publication number: WO 2024/016960

(56) References cited:
- EP-A1- 2 842 583
- WO-A1-2022/091833
- WO-A2-2006/083991
- CN-A- 1 672 739
- CN-A- 1 735 436
- CN-A- 105 169 496
- CN-A- 112 521 734
- CN-A- 115 154 664
- US-A1- 2003 215 483
- US-A1- 2003 215 483

## Description

### FIELD OF TECHNOLOGY

The present invention belongs to the field of biomedical materials and medical apparatuses and instruments, and specifically relates to a biodegradable ureteral stent and a preparation method therefor.

### BACKGROUND

Ureteral stents have been widely used in urological surgery, which are applicable to upper urinary tract surgery, lithotripsy using lithotripters, dilation of ureterostenosis, and other treatment processes, and can play important roles of draining urine and preventing ureterostenosis after implantation into ureters.

Existing clinically used ureteral stents are all non-degradable and are prepared from soft polyurethane elastomers or silicone rubber materials. The polyurethane stents are harder and easy to catheterize; and the silicone rubber stents are softer and have slightly poor catheterization performance. After catheterization of these two types of non-degradable ureteral stents, the hardness of the stents will remain unchanged. When a drainage function is completed, the ureteral stents need to be pulled out through an invasive operation, namely through a cystoscope, and patients will be in pain and are possible to have infections and other complications. Therefore, research and development of biodegradable ureteral stents have important clinical values.

According to the needs of clinical cases, the ureteral stents will have differences in drainage time. Short-term temporary drainage of urine usually takes about 1-2 weeks, such as mild ureteral injuries, simple stones, pre-stenting, etc.; and common temporary drainage usually takes 3-6 weeks. Thus, different degradation materials need to be used. However, common basic requirements for the stents include that they should have mechanical performance of elastic materials, and their degradation fragments can be completely excreted out of the body as soon as possible, otherwise the risk of a series of complications will be increased.

It has been reported (Lumiaho, J, J. Endourol. 1999, 13, 107-112; Laaksovirta, S Laurila M. et al. Jurol, 167:1527, 2002) that the ureteral stents are manufactured by using biodegradable polylactide (PLLA) or a lactide/glycolide copolymer (PLGA) as a raw material. However, the PLLA and PLGA materials are plastic, which lack softness, have harder degraded fragments, and have a great possibility of being embedded or getting stuck in the renal pelvis, leading to various complications.

A Chinese patent No. CN1672739A and a Chinese patent No. CN112516390A disclose a biodegradable ureteral stent, which relates to a glycolide-ε-caprolactone copolymer material and has mechanical performance of elastic materials within a certain composition proportion range. However, such copolymer formed by a soft chain monomer (ε-caprolactone) and a hard chain monomer (glycolide) usually becomes increasingly hard with degradation in water. This is because a hard chain structure in the material, such as a glycolide chain segment, usually has a crystallization trend in a degradation process, and thus, the material is easy to get stuck or remain in the renal pelvis and other parts. WO 2006/083991 A2 discloses a biodegradable ureteral stent comprising a terpolymer PEG, glycolide and epsilon-caprolactone; with barium sulfate dispersed in the terpolymer matrix.

In summary, the biodegradable materials obtained in the prior art cannot achieve the material properties which have not only mechanical performance similar to that of silicone rubbers and polyurethane elastic materials. but also can make its degradation fragments not to be stuck or not to remain in the renal pelvis. That is still a key point restricting the research progress in the art up to now and is also a primary reason why the degradable ureteral stents are currently not commercially available on the market.

### SUMMARY

In view of the above disadvantages of the prior art, the present invention provides a biodegradable ureteral stent. The stent has mechanical strength of elastic materials, higher initial hardness (or modulus) and convenience in catheterization, gradually becomes increasingly soft with degradation in urine, and is easier to be excreted out of the body.

Technical solutions of the present invention are as follows.

A biodegradable ureteral stent is prepared from a composite material, and the composite material is formed by blending at least a glycolide-ε-caprolactone copolymer, an ethylene oxide polymer, and barium sulfate with relative contents as follows:
1) a weight percentage content of the glycolide-ε-caprolactone copolymer is 47-80%;
2) a weight percentage content of the ethylene oxide polymer is 2-8%;
3) a weight percentage content of the barium sulfate is 18-45%; and
in the glycolide-ε-caprolactone copolymer, a weight percentage content of glycolide is 51-58%, and a weight percentage content of ε-caprolactone is 42-49%.

According to the ureteral stent of the present invention, the above purpose is realized by using the composite material formed by blending the degradable glycolide-ε-caprolactone copolymer, the ethylene oxide polymer, and the barium sulfate.

The present invention and related studies find that when the stent is harder, degraded fragments are easy to get stuck at the position of the renal pelvis. Thus, the softness and hardness of a substrate is a key influencing factor. Common degradable elastic materials, such as glycolide-ε-caprolactone copolymers, L-lactide/ε-caprolactone copolymers, etc., usually become increasingly hard in a degradation process, and degraded fragments or segments are more difficult to pass through narrow sites of the ureteral . However, studies of the present invention find that within an appropriate comonomer proportion range, by using an appropriate polymerization process, the glycolide-ε-caprolactone copolymer can gradually become soft with degradation in water or urine, and is an elastic material with good mechanical strength and suitable softness and hardness, and the appropriate comonomer proportion range is that the weight percentage content of the glycolide is 51-58%. When the weight percentage content of the glycolide is greater than 58%, the copolymer has an obvious crystallization trend and becomes hard in a degradation process; and when the weight percentage content of the glycolide is less than 51%, the copolymer has too poor mechanical performance and is too soft.

The present invention also finds that by adding a certain content of the ethylene oxide polymer, such as polyethylene glycol or polyoxyethylene, to materials of the glycolide-ε-caprolactone copolymer, the composite material can be further promoted to gradually become softer in a degradation process, smoother in surface, and easier to disintegrate and fracture, thereby being conducive to excretion of fractured fragments of the tubular stent.

According to the biodegradable ureteral stent of the present invention, the barium sulfate used is a commonly used radiopaque material in medical developer, which has good compatibility with the above materials and has a certain reinforcing effect.

According to the biodegradable ureteral stent of the present invention, an intrinsic viscosity of the glycolide-ε-caprolactone copolymer measured at 25±1°C in hexafluoroisopropanol at a concentration of 0.1 g/dl is 1.30-3.00 dl/g, and when the viscosity is greater, a degradation maintenance time is longer. Thus, a drainage time of the stent may be controlled by the intrinsic viscosity.

According to the biodegradable ureteral stent of the present invention, the ethylene oxide polymer includes polyethylene glycol, polyethylene glycol monomethyl ether, polyethylene glycol dimethyl ether, and polyoxyethylene.

As a preference, a weight proportion of the ethylene oxide polymer in the composite material is 2-8%, and when the content of the ethylene oxide polymer is higher, the composite material is softer and faster to degrade.

According to the biodegradable ureteral stent of the present invention, a molecular weight of the polyethylene glycol, the polyethylene glycol monomethyl ether, the polyethylene glycol dimethyl ether, or the polyoxyethylene is 1,000-1,000,000 Da.

As a preference, according to the biodegradable ureteral stent of the present invention, the molecular weight of the ethylene oxide polymer including the polyethylene glycol, the polyethylene glycol monomethyl ether, and the polyethylene glycol dimethyl ether is 5,000-40,000 Da.

As a preference, according to the biodegradable ureteral stent of the present invention, the ethylene oxide polymer is the polyoxyethylene, and the molecular weight is 50,000-400,000 Da.

According to the degradable ureteral stent of the present invention, a preparation method for the glycolide-ε-caprolactone copolymer includes:
under the protection of nitrogen, sequentially adding stannous octanoate with a mass ratio of 0.005-0.1%, ε-caprolactone, and glycolide to a reactor with a stirrer; raising the temperature of a reaction system from room temperature to 165-200°C within 30 minutes under stirring, maintaining the temperature for 18-30 hours, and then holding the system under vacuum for 1-4 hours; and transferring a copolymer out of the reactor, further breaking the copolymer, and placing the copolymer in a vacuum oven for vacuum drying at 50-110°C for 8-24 hours.

The biodegradable ureteral stent of the present invention is of a hollow circular tubular structure (1), a fixing structure (2) for preventing sliding is disposed at two ends or one end, the fixing structure is preferably in a circular tubular coil shape, a tube wall is also provided with a plurality of penetrating drainage side holes (3), and a tube diameter is 1.0-4.0 mm.

The biodegradable ureteral stent of the present invention is prepared by a melting extrusion method. The method specifically includes:
evenly mixing certain amounts of the glycolide-ε-caprolactone copolymer, the ethylene oxide polymer, and the medical developer, performing extrusion molding by an extruder at 120-160°C to obtain a degradable elastic tube material, subjecting the tube material to bending shaping at 50-80°C to form a fixing structure with a coil at one end or two ends, and then performing punching to obtain the biodegradable ureteral stent.

An initial modulus at 100% deformation of the degradable ureteral stent of the present invention is 2-10 MPa, and the modulus at 100% deformation after degradation is not greater than an initial value. An initial Shore hardness (A) of the material used is 70-95, and the Shore hardness (A) after degradation is not greater than an initial value.

According to the biodegradable ureteral stent of the present invention, during melting extrusion of a tube, various additives may be conventionally added to achieve different purposes, including but not limited to, a plasticizer, a lubricant, a dye, an antioxidant, an anti-hydrolysis agent, a melt thickener, a chain extender, a reinforcing agent, and a polymer modifier. These additives are conducive to improving the processing performance, degradation performance, surface performance, and mechanical performance of the stent.

Compared with the prior art, the present invention has the following beneficial effects.

In the present invention, by controlling the content of the glycolide monomer in the glycolide-ε-caprolactone copolymer, the copolymer has appropriate mechanical strength and softness and hardness and gradually becomes soft with prolonging of the degradation time. Meanwhile, a certain proportion of the ethylene oxide polymer is added, such that the polymer has a lower constant stretch modulus after degradation and is softer and smoother, thus is more suitable for preparing a biodegradable ureter stent.

### BRIEF DESCRIPTION OF THE DRAWINGS

FIG. 1 shows imaging observations of a biodegradable ureteral stent of the present invention excreted out of an animal, wherein in FIG. 1, (A) shows an X-ray image during implantation, (B) shows an X-ray image during fracturing, (C) shows an X-ray image during excretion into the bladder, and (D) shows excreted degraded fragments.
FIG. 2 is a schematic diagram of the biodegradable ureteral stent of the present invention.

### DESCRIPTION OF THE EMBODIMENTS

### Example 1 Preparation of a glycolide-ε-caprolactone copolymer 1

A 0.04% stannous octanoate catalyst, 570 g of an ε-caprolactone monomer (CL), and 580 g of a glycolide monomer (GA) were sequentially placed in a 3 L reactor. The temperature of a system was raised to 180°C within 25 minutes under the protection of nitrogen, a reaction was carried out for 25 hours at a stirring speed of 10-20 r/min, and the system was held under vacuum for 2 hours. A copolymer was transferred out of the reactor, further broken, and placed in a vacuum oven for vacuum drying at 90°C for 24 hours to obtain a glycolide-ε-caprolactone copolymer 1 (PGC1).

Weight percentage contents of the glycolide and the ε-caprolactone in the copolymer were determined by a ¹H nuclear magnetic spectrum, and hexafluoroisopropanol was used as a solvent.

The copolymer was prepared into a hexafluoroisopropanol solution at a concentration of 0.1 g/dl, and the intrinsic viscosity was tested by an Ubbelohde viscometer at 25°C.

The copolymer was prepared into a sheet with a thickness of 2 mm by a hot pressing molding method on a press vulcanizer at 140°C, and the Shore hardness (A) of the material was tested by a Shore hardness tester.

By using the same method, the copolymer was prepared into a dumbbell plate with a thickness of 2 mm, and the tensile strength at break and elongation at break of the material were tested on a universal mechanical tester at a speed of 200 mm/min.

An in vitro degradation experiment of the material was carried out in simulated urine at 37°C, and the Shore hardness (A) of the material was tested regularly.

### Example 2 Preparation of a glycolide-ε-caprolactone copolymer 2

A 0.02% stannous octanoate catalyst, 540 g of an ε-caprolactone monomer (CL), and 590 g of a glycolide monomer (GA) were sequentially placed in a 3 L reactor. The temperature of a system was raised to 170°C within 20 minutes under the protection of nitrogen, a reaction was carried out for 28 hours at a stirring speed of 10-20 r/min, and the system was held under vacuum for 1 hour. A copolymer was transferred out of the reactor, further broken, and placed in a vacuum oven for vacuum drying at 90°C for 24 hours to obtain a glycolide-ε-caprolactone copolymer 2 (PGC2).

Performance tests were carried out on the copolymer by using the method in Example 1. Test results are listed in Table 1.

### Example 3 Preparation of a glycolide-ε-caprolactone copolymer 3

A 0.03% stannous octanoate catalyst, 530 g of an ε-caprolactone monomer (CL), and 600 g of a glycolide monomer (GA) were sequentially placed in a 3 L reactor. The temperature of a system was raised to 190°C within 30 minutes under the protection of nitrogen, a reaction was carried out for 22 hours at a stirring speed of 10-20 r/min, and the system was held under vacuum for 4 hours. A copolymer was transferred out of the reactor, further broken, and placed in a vacuum oven for vacuum drying at 90°C for 24 hours to obtain a glycolide-ε-caprolactone copolymer 3 (PGC3).

Performance tests were carried out on the copolymer by using the method in Example 1. Test results are listed in Table 1.

### Example 4 Preparation of a glycolide-ε-caprolactone copolymer 4

A 0.05% stannous octanoate catalyst, 480 g of an ε-caprolactone monomer (CL), and 600 g of a glycolide monomer (GA) were sequentially placed in a 3 L reactor. The temperature of a system was raised to 195°C within 30 minutes under the protection of nitrogen, a reaction was carried out for 18 hours at a stirring speed of 10-20 r/min, and the system was held under vacuum for 4 hours. A copolymer was transferred out of the reactor, further broken, and placed in a vacuum oven for vacuum drying at 90°C for 24 hours to obtain a glycolide-ε-caprolactone copolymer 4 (PGC4).

Performance tests were carried out on the copolymer by using the method in Example 1. Test results are listed in Table 1.

### Example 5 Preparation of a glycolide-ε-caprolactone copolymer 5

A 0.01% stannous octanoate catalyst, 470 g of an ε-caprolactone monomer (CL), and 630 g of a glycolide monomer (GA) were sequentially placed in a 3 L reactor. The temperature of a system was raised to 190°C within 30 minutes under the protection of nitrogen, a reaction was carried out for 20 hours at a stirring speed of 10-20 r/min, and the system was held under vacuum for 2 hours. A copolymer was transferred out of the reactor, further broken, and placed in a vacuum oven for vacuum drying at 90°C for 24 hours to obtain a glycolide-ε-caprolactone copolymer 5 (PGC5).

Performance tests were carried out on the copolymer by using the method in Example 1. Test results are listed in Table 1.

### Example 6 Preparation of a glycolide-ε-caprolactone copolymer 6

A 0.01% stannous octanoate catalyst, 470 g of an ε-caprolactone monomer (CL), and 660 g of a glycolide monomer (GA) were sequentially placed in a 3 L reactor. The temperature of a system was raised to 184°C within 26 minutes under the protection of nitrogen, a reaction was carried out for 23 hours at a stirring speed of 10-20 r/min, and the system was held under vacuum for 3 hours. A copolymer was transferred out of the reactor, further broken, and placed in a vacuum oven for vacuum drying at 90°C for 24 hours to obtain a glycolide-ε-caprolactone copolymer 6 (PGC6).

Performance tests were carried out on the copolymer by using the method in Example 1. Test results are listed in Table 1.

**Table 1**

| Batch No. | GA/CL (weight%) | Intrinsic viscosity (g/dl) | Tensile strength (MPa) | Elongatio n at break (%) | Shore hardness before degradati on | Shore hardness after degradati on for 5 days | Shore hardness after degradation for 10 days |
|---|---|---|---|---|---|---|---|
| PGC1 | 51.2/48.8 | 2.25 | 10.2 | 1360 | 70 | 68 | 65 |
| PGC2 | 53.8/46.2 | 2.63 | 22.9 | 1201 | 82 | 77 | 74 |
| PGC3 | 54.1/45.9 | 1.92 | 22.3 | 1065 | 82 | 77 | 75 |
| PGC4 | 56.4/43.6 | 1.64 | 24.4 | 986 | 84 | 80 | 78 |
| PGC5 | 57.6/42.4 | 2.05 | 31.6 | 936 | 89 | 87 | 86 |
| PGC6 | 58.9/41.1 | 2.55 | 35.4 | 769 | 90 | 95 | 93 |

The above results indicate that when the content of glycolide in the glycolide-ε-caprolactone copolymers of the present invention is higher, the hardness is higher; when the weight content of glycolide is 51-58%, the Shore hardness (A) is equivalent to that of polyurethane (75-95) and silicone rubbers (50-70), and the copolymers are more suitable for use as ureteral stents; and degradation results in the simulated urine at 37°C show that the hardness of PGC 1 to PGC5 is decreased in a degradation process. The weight content of glycolide in PGC6 is greater than 58%, and the hardness is higher and is increased with increase of the degradation time. When the content of glycolide is lower than 51%, the tensile strength is lower.

### Example 7 Preparation of composite materials of glycolide-ε-caprolactone copolymers, polyethylene glycol, and barium sulfate

200 g of the glycolide-ε-caprolactone copolymers (PGC) prepared above, a certain amount of polyethylene glycol 20000 (PEG2) or polyethylene glycol 5000 (PEG5), and medical grade barium sulfate (Ba) were evenly mixed, and further blended and granulated through a twin-screw extruder at an extruder temperature of 120-150°C. Then, granules of resulting composite materials were prepared into sheets of the composite materials of the above three materials with a thickness of 2 mm by a vulcanizing press at 140°C, which were used for tensile strength and hardness tests. Dynamic friction coefficients of the composite materials were tested by a method specified by ASTM-D1894. Results are shown in Table 2.

**Table 2**

| Batch No. | Composition (weight%) | Tensile strength (MPa) | Shore hardness before degradation | Shore hardness after degradation for 5 days | Shore hardness after degradation for 10 days | Dynamic friction coefficien t |
|---|---|---|---|---|---|---|
| Composit e material 1 | 66PGC1 : 4PEG2:30Ba | 11.8 | 73 | 67 | 60 | 0.334 |
| Composit e material 2 | 65PGC3 : 8PEG2:27Ba | 18.2 | 78 | 68 | 62 | 0.193 |
| Composit e material 3 | 70PGC3 : 30Ba | 23.5 | 85 | 81 | 80 | 0.445 |
| Composit e material 4 | 61PGC5 : 2PEG2:37Ba | 28.3 | 92 | 83 | 76 | 0.378 |
| Composit e material 5 | 76PGC3 : 4PEG5:20Ba | 21.5 | 83 | 76 | 66 | 0.221 |
| Composit e material 6 | 60PGC3 : 5PEG5:35Ba | 19.7 | 81 | 70 | 61 | 0.283 |
| Composit e material 7 | 70PGC6 : 30Ba | 36.3 | 92 | 95 | 95 | 0.465 |
| Composit e material 8 | 65PGC6 : 5PEG5:30Ba | 31.0 | 89 | 90 | 88 | 0.288 |

The above results indicate that changes in the hardness of the composite materials containing PEG are obviously decreased in a degradation process in simulated urine with increase of the degradation time, and the higher content of PEG leads to a greater decrease of the hardness; the hardness of the composite material 3 without addition of PEG is decreased to a lower extent compared with that of the composite materials with addition of PEG; for the composite material 7 and the composite material 8, in the glycolide-ε-caprolactone copolymers used, the weight content of glycolide is greater than 58%, and thus the Shore hardness (A) is increased or slightly changed with prolonging of the degradation time; and the composite materials with addition of PEG have smaller dynamic friction coefficients, suggesting that surfaces are smoother.

### Example 8 Preparation of composite materials of glycolide-ε-caprolactone copolymers, polyoxyethylene, and barium sulfate

200 g of the glycolide-ε-caprolactone copolymers (PGC) prepared above, a certain amount of polyoxyethylene (PEO, molecular weight: 200,000 Da), and medical grade barium sulfate (Ba) were evenly mixed, and further blended and granulated through a twin-screw extruder at an extruder temperature of 120-140°C. Then, granules of resulting composite materials were prepared into sheets with a thickness of 2 mm by a vulcanizing press at 140°C, which were used for tensile strength and hardness tests. Dynamic friction coefficients of the composite materials were tested by a method specified by ASTM-D1894. Results are shown in Table 3.

**Table 3**

| Batch No. | Composition (weight%) | Tensile strength (MPa) | Shore hardness before degradati on | Shore hardness after degradati on for 5 days | Shore hardness after degradation for 10 days | Dynami c friction coefficie nt |
|---|---|---|---|---|---|---|
| Composite material 9 | 62PGC2 : 3PEO:35Ba | 21.8 | 85 | 74 | 67 | 0.377 |
| Composite material 10 | 60PGC2 : 5PEO:35Ba | 19.1 | 83 | 70 | 65 | 0.323 |
| Composite material 11 | 53PGC2 : 5PEO:42Ba | 18.4 | 85 | 75 | 67 | 0.353 |
| Composite material 12 | 73PGC2 : 20Ba | 23.7 | 85 | 76 | 75 | 0.431 |

Changes in the hardness of the above composite materials of the glycolide-ε-caprolactone copolymers, the polyoxyethylene, and the medical grade barium sulfate in a degradation process have similar rules, which are obviously decreased with increase of the degradation time. The higher content of polyoxyethylene leads to a greater decrease of the hardness. However, when the content of polyoxyethylene is too high, the strength is greatly reduced, which is not conducive to fixing. The composite materials with addition of polyoxyethylene have smaller dynamic friction coefficients, suggesting that surfaces are smoother.

### Example 9 Preparation of composite materials of glycolide-ε-caprolactone copolymers, polyethylene glycol, and bismuth subcarbonate

200 g of the glycolide-ε-caprolactone copolymers (PGC) prepared above, a certain amount of polyethylene glycol 20000 (PEG2) or polyethylene glycol 5000 (PEG5), and medical bismuth subcarbonate (Bi) were evenly mixed, and further blended and granulated through a twin-screw extruder at an extruder temperature of 120-140°C. Then, granules of resulting composite materials were prepared into sheets of the composite materials of the above three materials with a thickness of 2 mm by a vulcanizing press at 140°C, which were used for tensile strength and hardness tests. Dynamic friction coefficients of the composite materials were tested by a method specified by ASTM-D1894. Results are shown in Table 4.

**Table 4**

| Batch No. | Composition (weight%) | Tensile strength (MPa) | Shore hardness before degradati on | Shore hardness after degradati on for 5 days | Dynami c friction coefficie nt |
|---|---|---|---|---|---|
| Composite material 13 | 77PGC2 : 5PEG2:18Bi | 14.4 | 75 | 64 | 0.239 |
| Composite material 14 | 80PGC2 : 8PEG5: 12Bi | 12.1 | 63 | 51 | 0.156 |

Properties and change rules of the above composite materials of the glycolide-ε-caprolactone copolymers, the polyethylene glycol, and the bismuth subcarbonate are similar to those of the above composite materials, that is, changes in the hardness of the composite materials in a degradation process in simulated urine are obviously decreased with increase of the degradation time, the composite materials with addition of PEG have smaller dynamic friction coefficients, and the above rules are not related to the type of a developer used.

### Example 10 Preparation of degradable ureteral stents

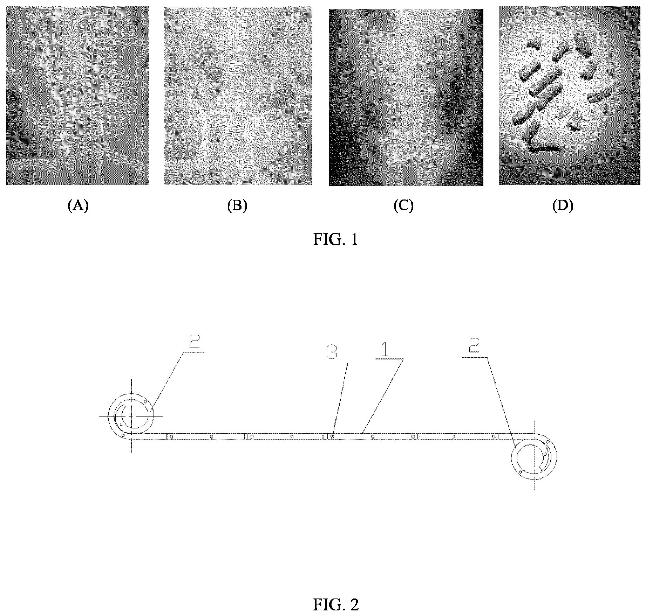

The composite materials prepared in the above examples were subjected to extrusion molding by an extruder at 120-150°C to obtain a degradable elastic tube body 1, respectively, wherein the tube body 1 had an outer diameter of 2 mm and an inner diameter of 1.1 mm. The tube body was subjected to bending shaping at 50-80°C to form a fixing structure with a coiled tubular coil 2 formed at one end or two ends. Then, the tube body was subjected to punching (longitudinal aperture: 1.0 mm) at an equal distance (hole distance: 50 mm) by a punching device to form drainage holes 3, so as to obtain a degradable ureteral stent shown in FIG. 2.

On a universal mechanical tester, the tensile strength at break and constant stretch modulus (tensile strength at the elongation of 100%, same as the Shore hardness for characterizing the softness and hardness of a material) of materials were tested at a speed of 200 mm/min, and changes in the constant stretch modulus in an in vitro degradation process in simulated urine at 37°C were tested. Results are shown in Table 5.

### Example 11 In vivo degradation experiment of degradable ureteral stents in animals

After miniature pigs were selected and subjected to general anesthesia, ureteral stents were implanted into left and right ureters of the miniature pigs by a ureteroscopy method, in which tubular coils at upper ends were fixed in the renal pelvis, and tubular coils at lower ends were fixed in the bladder. Whether the ureteral stents with the situations of slipping, fracturing, and excretion at different time points were observed by an X-ray after ureteroscopy. The constant stretch modulus of the stents was tested by a universal mechanical tester at different time points to characterize changes in the softness and hardness. Results are shown in Table 5.

**Table 5**

| Ureter al stent | Material used | Tensile strength (MPa) | Modulus before degradation (MPa) | Modulus after degradation for 8 days (MPa) | Fracture time (day) | Complete excretion time (day) |
|---|---|---|---|---|---|---|
| 1 | Composite material 1 | 13.5 | 3.1 | 1.3 | 8 | 13 |
| 2 | Composite material 6 | 22.3 | 4.8 | 3.6 | 12 | 18 |
| 3 | Composite material 9 | 24.4 | 5.9 | 3.8 | 17 | 25 |
| 4 | Composite material 3 | 25.9 | 5.8 | 5.0 | 14 | 40 |
| 5 | Composite material 12 | 24.9 | 5.4 | 4.8 | 15 | 45 |
| 6 | Commercial silicone rubber | 9.9 | 2.7 | 2.6 | | |
| 7 | Commercial polyurethane | 25.7 | 7.8 | 7.8 | | |

The above results show that the ureteral stents 1, 2, and 3 are added with the ethylene oxide polymer, while the ureteral stents 4 and 5 are not added with the ethylene oxide polymer. All the degradable stents can be completely excreted from the urinary system of animals, and the fracture time depends on the molecular weight of materials, the proportions of comonomers, the amount of the ethylene oxide polymer added, and other factors, including process conditions during processing, etc. However, since the ureteral stents 1, 2, and 3 with addition of the ethylene oxide polymer have lower constant stretch modulus after degradation and are softer and smoother in surface, these ureteral stents have a shorter complete excretion time in vivo than the ureteral stents 4 and 5 without addition of the ethylene oxide polymer, and thus have a smaller possibility of incrustation in vivo. The various groups of ureteral stents have certain but not large differences in initial modulus, and can all undergo catheterization smoothly. The modulus of a commercial silicone rubber stent and a polyurethane stent is not changed with the degradation time. The tensile strength and modulus of the degradable ureteral stents of the present invention are between those of the silicone rubber stent and the polyurethane stent, and are closer to those of the polyurethane stent.

FIG. 1 shows imaging observations of the biodegradable ureteral stent of the present invention excreted out of an animal. The biodegradable ureteral stent can be smoothly excreted from the renal pelvis, the ureters, and the bladder without residual fragments after degradation.

## Claims

1. A biodegradable ureteral stent, **characterized in that** the biodegradable ureteral stent is prepared from a composite material, the material is formed by blending at least a glycolide-ε-caprolactone copolymer, an ethylene oxide polymer, and barium sulfate with relative contents as follows:
1) a weight percentage content of the glycolide-ε-caprolactone copolymer is 47-80%;
2) a weight percentage content of the ethylene oxide polymer is 2-8%;
3) a weight percentage content of the barium sulfate is 18-45%; and
in the glycolide-ε-caprolactone copolymer, a weight percentage content of glycolide is 51-58%, and a weight percentage content of ε-caprolactone is 42-49%.

2. The biodegradable ureteral stent according to claim 1, **characterized in that** an intrinsic viscosity of the glycolide-ε-caprolactone copolymer measured at 25±1°C in hexafluoroisopropanol at a concentration of 0.1 g/dl is 1.30-3.00 dl/g.

3. The biodegradable ureteral stent according to claim 1, **characterized in that** the ethylene oxide polymer comprises polyethylene glycol, polyethylene glycol monomethyl ether, polyethylene glycol dimethyl ether, polyoxyethylene, and an ethylene oxide copolymer; and
a molecular weight of the polyethylene glycol, the polyethylene glycol monomethyl ether, the polyethylene glycol dimethyl ether, or the polyoxyethylene is 1,000-1,000,000 Da.

4. The biodegradable ureteral stent according to claim 3, **characterized in that** the ethylene oxide polymer is the polyethylene glycol or the polyethylene glycol monomethyl ether, and the molecular weight is 5,000-40,000 Da; and alternatively,
the ethylene oxide polymer is the polyoxyethylene, and the molecular weight is 50,000-400,000 Da.

5. The biodegradable ureteral stent according to claim 1, **characterized in that** the degradable ureteral stent is of a hollow circular tubular structure (1), a fixing structure (2) for preventing sliding is disposed at two ends or one end, a tube wall is also provided with a plurality of penetrating drainage side holes (3), and an outer diameter of a tube is 1.0-4.0 mm.

6. The biodegradable ureteral stent according to claim 1, **characterized in that** an initial modulus at 100% deformation of the degradable ureteral stent is 2-10 MPa, and the modulus at 100% deformation after degradation is not greater than an initial value; and an initial Shore hardness (A) of the material used is 70-95, and the Shore hardness (A) after degradation is not greater than an initial value.

7. The biodegradable ureteral stent according to claim 1 or 5, **characterized in that** various additives are also able to be added during melting extrusion of the biodegradable ureteral stent, comprising a plasticizer, a lubricant, a dye, an antioxidant, an anti-hydrolysis agent, a melt thickener, a chain extender, a reinforcing agent, and a polymer modifier.

8. A preparation method for the biodegradable ureteral stent according to any one of claims 1-7, **characterized in that** the preparation method comprises: evenly mixing the glycolide-ε-caprolactone copolymer, the ethylene oxide polymer, and the medical developer, performing extrusion molding by an extruder at 120-160°C to obtain a degradable elastic tube material, subjecting the tube material to bending shaping at 50-80°C to form a fixing structure with a tubular coil at one end or two ends, and then performing punching to obtain the degradable ureteral stent.

9. The preparation method for the biodegradable ureteral stent according to claim 8, **characterized in that** the preparation method further comprises a preparation process of the glycolide-ε-caprolactone copolymer:
under the protection of nitrogen, sequentially adding stannous octanoate with a mass ratio of 0.005-0.1%, ε-caprolactone, and glycolide to a reactor with a stirrer; raising the temperature of a reaction system from room temperature to 165-200°C within 30 minutes under stirring, maintaining the temperature for 18-30 hours, and then holding the system under vacuum for 1-4 hours; and transferring a copolymer out of the reactor, further breaking the copolymer, and placing the copolymer in a vacuum oven for vacuum drying at 50-110°C for 8-24 hours.

## Patentansprüche

1. Biologisch abbaubarer Harnleiterstent, **dadurch gekennzeichnet, dass** der biologisch abbaubare Harnleiterstent aus einem Verbundmaterial hergestellt ist, wobei das Material durch Mischen wenigstens eines Glycolid-ε-Caprolacton-Copolymers, eines Ethylenoxidpolymers und Bariumsulfat mit relativen Gehalten wie folgt gebildet ist:
1) ein Gewichtsprozentgehalt des Glycolid-ε-Caprolacton-Copolymers beträgt 47-80 %;
2) ein Gewichtsprozentgehalt des Ethylenoxidpolymers beträgt 2-8 %;
3) ein Gewichtsprozentgehalt des Bariumsulfats beträgt 18-45 %; und
in dem Glycolid-ε-Caprolacton-Copolymer beträgt ein Gewichtsprozentgehalt von Glycolid 51-58 %, und ein Gewichtsprozentgehalt von ε-Caprolacton beträgt 42-49 %.

2. Biologisch abbaubarer Harnleiterstent nach Anspruch 1, **dadurch gekennzeichnet, dass** eine bei 25 ± 1 °C in Hexafluorisopropanol bei einer Konzentration von 0,1 g/dl gemessene intrinsische Viskosität des Glycolid-ε-Caprolacton-Copolymers 1,30-3,00 dl/g beträgt.

3. Biologisch abbaubarer Harnleiterstent nach Anspruch 1, **dadurch gekennzeichnet, dass** das Ethylenoxidpolymer Polyethylenglycol, Polyethylenglycolmonomethylether, Polyethylenglycoldimethylether, Polyoxyethylen und ein Ethylenoxid-Copolymer umfasst; und
ein Molekulargewicht des Polyethylenglycols, des Polyethylenglycolmonomethylethers, des Polyethylenglycoldimethylethers oder des Polyoxyethylens 1.000-1.000.000 Da beträgt.

4. Biologisch abbaubarer Harnleiterstent nach Anspruch 3, **dadurch gekennzeichnet, dass** das Ethylenoxidpolymer das Polyethylenglycol oder der Polyethylenglycolmonomethylether ist und das Molekulargewicht 5.000-40.000 Da beträgt; und alternativ
das Ethylenoxidpolymer das Polyoxyethylen ist und das Molekulargewicht 50.000-400.000 Da beträgt.

5. Biologisch abbaubarer Harnleiterstent nach Anspruch 1, **dadurch gekennzeichnet, dass** der abbaubare Harnleiterstent eine hohle, kreisförmige rohrförmige Struktur (1) aufweist, an zwei Enden oder einem Ende eine Fixierstruktur (2) zum Verhindern eines Gleitens angeordnet ist, eine Rohrwand ferner mit mehreren durchgehenden Drainage-Seitenlöchern (3) versehen ist und ein Außendurchmesser eines Rohrs 1,0-4,0 mm beträgt.

6. Biologisch abbaubarer Harnleiterstent nach Anspruch 1, **dadurch gekennzeichnet, dass** ein Anfangsmodul bei 100 % Verformung des abbaubaren Harnleiterstents 2-10 MPa beträgt und der Modul bei 100 % Verformung nach Abbau nicht größer als ein Anfangswert ist; und eine anfängliche Shore-Härte (A) des verwendeten Materials 70-95 beträgt und die Shore-Härte (A) nach Abbau nicht größer als ein Anfangswert ist.

7. Biologisch abbaubarer Harnleiterstent nach Anspruch 1 oder 5, **dadurch gekennzeichnet, dass** während einer Schmelzextrusion des biologisch abbaubaren Harnleiterstents ferner verschiedene Additive zugesetzt werden können, umfassend einen Weichmacher, ein Schmiermittel, einen Farbstoff, einen Antioxidator, ein Antihydrolysemittel, einen Schmelzverdicker, einen Kettenverlängerer, ein Verstärkungsmittel und einen Polymermodifikator.

8. Herstellungsverfahren für den biologisch abbaubaren Harnleiterstent nach einem der Ansprüche 1-7, **dadurch gekennzeichnet, dass** das Herstellungsverfahren umfasst: gleichmäßiges Mischen des Glycolid-ε-Caprolacton-Copolymers, des Ethylenoxidpolymers und des medizinischen Kontrastmittels, Durchführen eines Extrusionsformens mittels eines Extruders bei 120-160 °C, um ein abbaubares elastisches Rohrmaterial zu erhalten, Unterziehen des Rohrmaterials einer Biegeformgebung bei 50-80 °C, um eine Fixierstruktur mit einer rohrförmigen Spirale an einem Ende oder an zwei Enden zu bilden, und anschließendes Durchführen eines Stanzens, um den abbaubaren Harnleiterstent zu erhalten.

9. Herstellungsverfahren für den biologisch abbaubaren Harnleiterstent nach Anspruch 8, **dadurch gekennzeichnet, dass** das Herstellungsverfahren ferner einen Herstellungsprozess des Glycolid-ε-Caprolacton-Copolymers umfasst: unter Schutz von Stickstoff, sequenzielles Zugeben von Zinn(II)-octanoat mit einem Massenverhältnis von 0,005-0,1 %, ε-Caprolacton und Glycolid in einen Reaktor mit einem Rührer; Erhöhen der Temperatur eines Reaktionssystems von Raumtemperatur auf 165-200 °C innerhalb von 30 Minuten unter Rühren, Halten der Temperatur für 18-30 Stunden und anschließendes Halten des Systems unter Vakuum für 1-4 Stunden; und Überführen eines Copolymers aus dem Reaktor, weiteres Zerkleinern des Copolymers und Platzieren des Copolymers in einem Vakuumofen zum Vakuumtrocknen bei 50-110 °C für 8-24 Stunden.

## Revendications

1. Stent urétéral biodégradable, **caractérisé en ce que** le stent urétéral biodégradable est préparé à partir d'un matériau composite, le matériau est formé par mélange d'au moins un copolymère de glycolide-ε-caprolactone, d'un polymère d'oxyde d'éthylène et de sulfate de baryum, dans les proportions suivantes :
1) une teneur en pourcentage en poids du copolymère glycolide-ε-caprolactone est de 47-80 % ;
2) une teneur en pourcentage en poids du polymère d'oxyde d'éthylène est de 2-8 % ;
3) une teneur en pourcentage en poids du sulfate de baryum est de 18-45 % ; et
dans le copolymère de glycolide-ε-caprolactone, une teneur en pourcentage en poids de glycolide est de 51-58 %, et une teneur en pourcentage en poids de ε-caprolactone est de 42-49 %.

2. Stent urétéral biodégradable selon la revendication 1, **caractérisé en ce qu'**une viscosité intrinsèque du copolymère de glycolide-ε-caprolactone, mesurée à 25±1 °C dans de l'hexafluoroisopropanol à une concentration de 0,1 g/dl, est de 1,30-3,00 dl/g.

3. Stent urétéral biodégradable selon la revendication 1, **caractérisé en ce que** le polymère d'oxyde d'éthylène comprend du polyéthylène glycol, du monométhyléther de polyéthylène glycol, du diméthyléther de polyéthylène glycol, du polyoxyéthylène et un copolymère d'oxyde d'éthylène ; et
une masse moléculaire du polyéthylène glycol, du monométhyléther de polyéthylène glycol, du diméthyléther de polyéthylène glycol ou du polyoxyéthylène est de 1 000-1 000 000 Da.

4. Stent urétéral biodégradable selon la revendication 3, **caractérisé en ce que** le polymère d'oxyde d'éthylène est le polyéthylène glycol ou le monométhyléther de polyéthylène glycol, et la masse moléculaire est de 5 000-40 000 Da ; et, en variante,
le polymère d'oxyde d'éthylène est le polyoxyéthylène, et la masse moléculaire est de 50 000-400 000 Da.

5. Stent urétéral biodégradable selon la revendication 1, **caractérisé en ce que** le stent urétéral biodégradable présente une structure tubulaire circulaire creuse (1), une structure de fixation (2) destinée à empêcher tout glissement est disposée aux deux extrémités ou à une seule extrémité, une paroi de tube est également dotée d'une pluralité de trous latéraux d'évacuation traversants (3), et un diamètre extérieur d'un tube est de 1,0-4,0 mm.

6. Stent urétéral biodégradable selon la revendication 1, **caractérisé en ce qu'**un module initial à 100 % de déformation du stent urétéral biodégradable est de 2-10 MPa, et le module à 100 % de déformation après dégradation est inférieur ou égal à une valeur initiale ; et une dureté Shore (A) initiale du matériau utilisé est de 70-95, et la dureté Shore (A) après dégradation est inférieure ou égale à une valeur initiale.

7. Stent urétéral biodégradable selon la revendication 1 ou 5, **caractérisé en ce que** divers additifs peuvent également être incorporés lors de l'extrusion à l'état fondu du stent urétéral biodégradable, comprenant un plastifiant, un lubrifiant, un colorant, un antioxydant, un agent anti-hydrolyse, un épaississant de matière fondue, un agent d'allongement de chaîne, un agent de renforcement et un modificateur de polymère.

8. Procédé de préparation d'un stent urétéral biodégradable selon l'une quelconque des revendications 1-7, **caractérisé en ce que** le procédé de préparation comprend : le mélange homogène du copolymère de glycolide-ε-caprolactone, du polymère d'oxyde d'éthylène et du développateur médical, la réalisation d'un moulage par extrusion à l'aide d'une extrudeuse à 120-160 °C afin d'obtenir un matériau tubulaire élastique dégradable, la soumission du matériau tubulaire à un formage par cintrage à 50-80 °C pour former une structure de fixation comportant une spirale tubulaire à une extrémité ou à deux extrémités, puis la réalisation d'un poinçonnage pour obtenir le stent urétéral dégradable.

9. Procédé de préparation d'un stent urétéral biodégradable selon la revendication 8, **caractérisé en ce que** le procédé de préparation comprend en outre un processus de préparation du copolymère de glycolide-ε-caprolactone :
sous atmosphère d'azote, l'ajout successif d'octanoate stanneux selon un rapport massique de 0,005-0,1 %, d'ε-caprolactone et de glycolide dans un réacteur muni d'un agitateur ; l'élévation de la température d'un système réactionnel de la température ambiante à 165-200 °C en 30 minutes sous agitation, la conservation de la température pendant 18-30 heures, puis le maintien du système sous vide pendant 1-4 heures ; et la sortie du copolymère du réacteur, la décomposition supplémentaire du copolymère, puis le placement dans un four sous vide pour un séchage sous vide à 50-110 °C pendant 8-24 heures.
